# EUROPÄISCHE PATENTANMELDUNG

(11) **EP 0 816 354 A1**
(43) Veröffentlichungstag der Anmeldung: **07.01.1998**
(21) Anmeldenummer: 97109555.9
(22) Anmeldetag: 12.06.1997
(51) Int. Cl.: C07D 319/06, C07D 319/08, C07D 493/10, C07F 9/00, C07C 309/00, C07C 301/00, C07C 305/00

(54) **Verfahren zur Herstellung und Stabilisierung von aliphatischen Sechsringcarbonaten**

(30) Priorität: 25.06.1996 DE 19625265; 22.07.1996 DE 19629462
(71) Anmelder: BAYER AG, 51368 Leverkusen (DE)
(72) Erfinder: Buysch, Hans-Josef, Dr., 47809 Krefeld (DE); Fengler, Gerd, Dr., 47802 Krefeld (DE); Neumann, Karl-Heinz, Dr., 53757 Sankt Augustin (DE); Wagner, Paul, Dr., 40597 Düsseldorf (DE); Melchiors, Martin, Dr., 52064 Aachen (DE); Hovestadt, Wieland, Dr., 47800 Krefeld (DE)

(57) **Zusammenfassung**

Die Erfindung betrifft ein Verfahren zur Herstellung von Sechsringcarbonaten der Formel 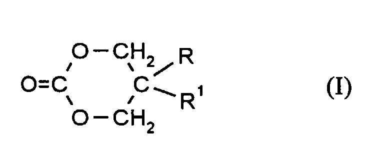 durch Umesterung von 1,3-Propandiol-Verbindungen mit Kohlensäureestern und anschließende destillative Depolymerisation des zunächst entstehenden Oligo- bzw. Polycarbonats, worin man sowohl für die Umesterung als auch für die Depolymerisation einen oder mehrere Katalysatoren aus der Gruppe der Verbindungen von Sn, Ti oder Zr einsetzt und gegebenenfalls den Destillationsrückstand mindestens teilweise in die Umesterung zurückführt. Solche Sechsringcarbonate können weiterhin durch Zusatz Kleiner Mengen von Säuren des Schwefels oder Phosphors oder deren Estern oder Salzen gegen unerwünschte Polymerisation stabilisiert werden.

## Beschreibung

Die vorliegende Erfindung betrifft die Herstellung von cyclischen Sechsringcarbonaten durch Umesterung von 1,3-Propandiol-Verbindungen mit Kohlensäureestern und nachfolgende destillative Depolymerisation des zunächst entstehenden Oligo- bzw. Polycarbonats, wobei in beiden Stufen in Gegenwart von Katalysatoren aus der Gruppe der Verbindungen von Sn, Ti und Zr gearbeitet wird und gegebenenfalls der Destillationsrückstand der Depolymerisation mindestens teilweise in die Umesterung züruckgeführt wird. Die erfindungsgemäß herstellbaren cyclischen Carbonate können weiterhin gegen unerwünschte Polymerisation stabilisiert werden. Die vorliegende Erfindung betrifft daher weiterhin so stabilisierte cyclische Carbonate mit geringen Mengen von Säuren des Schwefels oder Phosphors oder deren Estern oder Salzen, die bei thermischer Belastung eine deutlich geringere Polymerisationsneigung aufweisen als nicht stabilisierte.

Bekanntlich lassen sich Sechsringcarbonate (I) dadurch gewinnen, daß man 1,3-Propandiol-Verbindungen (II) mit Kohlensäureestern (III) unter Abspaltung der Hydroxyverbindungen R³OH umestert und die dabei erhaltenen Oligo- oder Polycarbonate unter den Bedingungen einer Vakuumdestillation zu (I) depolymerisiert, (I) abtrennt und gewinnt. Derartige Verfahren sind, z.B. in US-PS 45 01 905 und US-PS 44 40 937 beschrieben. Zur Umesterung werden hierbei stark alkalische Katalysatoren aus der Gruppe der Oxide, Hydroxide, Alkoholate, Carboxylate und Carbonate von Alkalimetallen (bevorzugt Na und K), Aluminium, Thallium oder Blei eingesetzt. Bei der Depolymerisation erhält man je nach den angewandten Bedingungen, wie Druck, Temperatur und Reaktionsapparatur und der Menge und Art der verwendeten Katalysatoren einen mehr oder weniger großen Destillationsrückstand von etwa 25 bis günstigstenfalls 5 % des eingesetzten Oligo- oder Polycarbonates, der entsorgt werden muß und die Ausbeute an (I) dementsprechend mindert.

Es wurde nun gefunden, daß ein Teil der Nachteile durch eine andere Reaktionsführung vermieden werden kann, bei der sowohl in der Stufe der Umesterung als auch in der Stufe der Depolymerisation in Gegenwart von Verbindungen von Sn, Ti oder Zr gearbeitet wird. Zur weiteren Verfahrensverbesserung kann man den Destillationsrückstand der Depolymerisation ganz oder wenigstens teilweise wieder in die Umesterung von (II) mit (III) einsetzen, somit die Einsatzmenge an (II) und (III) für eine bestimmte Menge an (I) verkleinern und die Ausbeute an (I), bezogen auf eingesetztes (II) und (III), entsprechend erhöhen.

Bekanntlich kann man cyclische Carbonate in Gegenwart verschiedener Katalysatoren in Polycarbonate überführen. Dazu sei auf folgende Schriften verwiesen: US 4 501 905, US 45 68 755, EP 236 862, US 4 707 539, EP-188 204, US 4 252 750. Solche Polymerisationen laufen zwar weit langsamer, aber doch merklich auch schon ohne zusätzlichen Katalysator ab, wenn man cyclische Carbonate über längere Zeit thermisch belastet, beispielsweise sie im geschmolzenen Zustand halt. An sich wirkt sich eine solche Polymerisation nicht nachteilig aus, wenn eine Schmelze ohnehin einer Polycarbonatsynthese zugeführt werden soll. Sehr störend macht sich eine solche Polymerisationsneigung jedoch bemerkbar, wenn ein cyclisches Carbonat beispielsweise schmelzflüssig über eine längere Zeit gelagert werden soll, um dann aber andere Polymerreaktionen als die Eigenpolymerisation durchzuführen. Der entstandene Polymeranteil nimmt dann auch an solchen anderen Reaktionen teil, verschlechtert jedoch deren Ergebnis, mindert die Reproduzierbarkeit und kann sogar unbrauchbare Produkte verursachen. Auch dann, wenn Copolymerisationen verschiedener Dioxanone durchgeführt werden sollen, beeinträchtigt eine Vorpolymerisation einzelner Dioxanone das Ergebnis einer Copolymerisation in nicht vorhersehbarer Weise und kompliziert das Polymerisationsverfahren in jedem Fall.

Besonders ausgeprägte Neigung zur Oligomerisierung und Polymerisation weisen die cyclischen Carbonate der Trimethylolalkane auf also die 5-Methylol-dioxanone der Formel (VI) 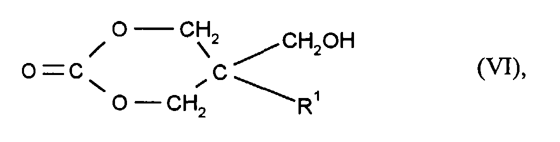 worin
- R¹: C₁-C₆-Alkyl, Cyclohexyl oder C₆-C₁₂-Aryl bedeutet.

Genannt sei vor allem das Carbonat des Trimethylolpropans, aus dem interessante Lackrohstoffe hergestellt werden können (vergl. EP 600 417, EP 665 260 und EP 703 230).

Überraschend wurde nun ergänzend gefunden, daß geringe Zusätze von Säuren des Schwefels und/oder des Phosphors oder deren Ester oder Salze die Polymerisationsgeschwindigkeit der cyclischen Carbonate deutlich verlangsamen. Die Erfindung betrifft daher weiterhin Gemische von cyclischen Carbonaten mit Säuren des Schwefels und/oder Phosphors und/oder deren Estern oder Salzen in den weiter unten angegebenen Mengenverhältnissen.

Die Erfindung betrifft ein Verfahren zur Herstellung von gegebenenfalls stabilisierten cyclischen Carbonaten der Formel (I) 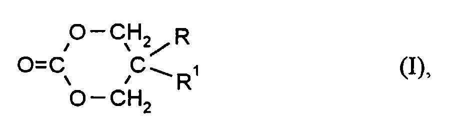 in der
- R und R¹: gleich oder verschieden sind und H, geradkettiges oder verzweigtes C₁-C₆-Alkyl, C₃-C₇-Cycloalkyl oder -CH₂-OR² darstellen, worin R² für H, C₁-C₄-Alkyl, Allyl, Methallyl oder Benzyl steht und worin zwei R²-Gruppen gemeinsam -CH₂-, -CH(CH₃)-, -CH(C₂H₅)-, -CH(C₆H₅)- oder -CH(C₃H₇)- sein können, wobei R¹ zusätzlich C₆-C₁₂-Aryl bedeuten kann und wobei weiterhin R und R¹ gemeinsam mit dem C-Atom, das sie substituieren, einen Cyclopentan-, Cyclohexan-, Cycloheptan-, Oxetan-, Tetrahydrofuran-, Tetrahydropyran oder Dioxanring bilden können,
durch Umesterung von 1,3-Propandiol-Verbindungen der Formel (II) 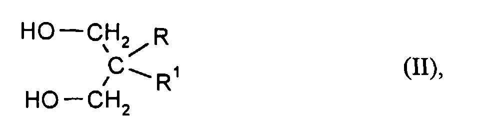 in der R und R¹ die oben angegebene Bedeutung haben,
mit Kohlensäureestern der Formel (III) 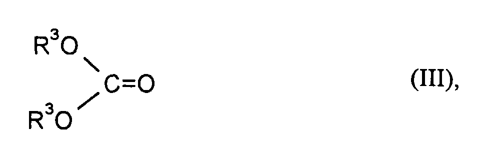 in der R³ C₁-C₄-Alkyl oder C₆-C₁₀-Aryl bedeutet,
unter Abspaltung von R³OH zu Oligo- oder Polycarbonaten und durch nachfolgende destillative Depolymerisation der so erhaltenen Oligo- oder Polycarbonate zu cyclischen Carbonaten (I), das dadurch gekennzeichnet ist, daß man sowohl in der Stufe der Umesterung als auch in der nachfolgenden destillativen Depolymerisation in Gegenwart eines oder mehrerer Katalysatoren aus der Gruppe der Verbindungen von Zinn, Titan und Zirkon in einer Menge von 0,001-5 Gew.-%, bevorzugt in einer Menge von 0,03-0,1 Gew.-%, bezogen auf die Gesamtmenge von (II) und (III), arbeitet, wobei zur Umesterung bei einer Temperatur von 120-180°C, bevorzugt von 150-170°C und zur Depolymerisation bei einer Temperatur von 150-280°C, bevorzugt 190-250°C, besonders bevorzugt von 200-240°C gearbeitet wird und die Temperatur zur Depolymerisation 30-150°C über der der Umesterung liegt, und man gegebenenfalls den bei der Depolymerisation erhaltenen Destillationsrückstand zu 50-100% seiner Menge in die Umsetzung von (II) mit (III) zurückführt. und wobei man den cyclischen Carbonaten (I) nach der Depolymerisation zur Stabilisierung gegen unerwünschte Polymerisation eine oder mehrere Verbindungen aus der Gruppe der Säuren des Schwefels und des Phosphors sowie deren Estern und Salzen in einer Menge von 0,001 - 5 Gew.-%, bezogen auf die Menge von (I), zusetzen kann.

Die Erfindung betrifft auch Gemische von cyclischen Carbonaten der Formel (I) mit mindestens einer Verbindung aus der Gruppe der Säuren des Schwefels und des Phosphors sowie deren Estern und Salzen in Mengen von 0,001 bis 5 Gew.-% der genannten Schwefel- bzw. Phosphorverbindungen, bezogen auf die Menge der cyclischen Carbonate, sowie gegegebenenfalls mit einem zusätzlichen Gehalt an einem oder mehreren Lösungsmitteln aus der Gruppe der Ether, Ester, Ketone, Nitrile, Amide, Lactame und Lactone, bevorzugt aus der Gruppe der Ester, Amide, Lactame und Lactone, in einer Menge von 2 bis 500 %, bevorzugt 3 bis 300 %, besonders bevorzugt 5 bis 200 % des Gesamtgewichts von cyclischen Carbonaten und S- bzw. P-Verbindung.

Solche Gemische enthalten bevorzugt cyclische Carbonate der Formel (VI).

Geradkettiges oder verzweigtes C₁-C₆-Alkyl ist beispielsweise Methyl, Ethyl, Propyl, i-Propyl, Butyl, i-Butyl, Pentyl und seine Isomeren oder Hexyl und seine Isomeren. Bevorzugtes Alkyl hat 1-4 C-Atome.

C₃-C₇-Cycloalkyl ist beispielsweise Cyclopropyl, Cyclobutyl, Cyclopentyl, Cyclohexyl oder Cycloheptyl, bevorzugt Cyclohexyl.

Alkyl und Cycloalkyl können durch OH und/oder OCH₃ und/oder OC₂H₅ substituiert sein.

C₆-C₁₂-Aryl ist beispielsweise Phenyl, Tolyl, Xylyl, Chlorphenyl, Naphthyl oder Biphenyl, bevorzugtes Aryl ist Phenyl, Tolyl und Chlorphenyl; besonders bevorzugt ist Phenyl.

In bevorzugter Weise treten die Substituenten R¹⁰ und R¹¹ an die Stelle von R und R¹ R¹⁰ und R¹¹ sind gleich oder verschieden und bedeuten H, CH₃, C₂H₅, oder -CH₂-OR², wobei R² die obige Bedeutung hat.

Genannt als (II) seien beispielsweise Propandiol-1,3, 2-Ethylpropandiol-1,3, 2,2-Dimethyl-propandiol-1,3, 1,1-Dimethylol-cyclohexan, Trimethylolethan, Trimethylolpropan, Pentaerythrit, Trimethylpropan-mono-methylether, Trimethylol-propan-monoethylether, Trimethylolpropan-mono-benzylether, Trimethylpropan-mono-allylether und Pentaerythrit-formal der Formel (IV) und 3,3-Dimethyloloxetan der Formel (V)

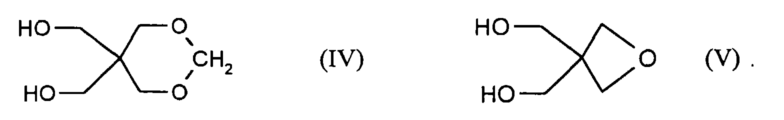

Geeignete Kohlensäureester sind solche der Formel (III), in der R³ C₁-C₄-Alkyl oder Aryl bedeutet, also Dimethyl-, Diethyl-, Dipropyl-, Diisopropyl-, Dibutyl-, Diisobutyl-, Diphenyl- und Dikresylcarbonat und Dinaphthylcarbonat.

Die Umesterung der Einsatzprodukte gemäß (II) und (III) vollzieht sich nach den in US 4 501 905 und US 4 440 937 beschriebenen Verfahren und wird im Temperaturbereich von 60-250°C, bei Normaldruck oder bei vermindertem Druck, bevorzugt im Bereich von 0,001-140 mbar und in Gegenwart von Katalysatoren aus der Gruppe von Alkalien, Zinn-, Titanverbindungen, die in US 4 501 905 und US 4 440 937 angegeben sind, durchgeführt.

Die nachfolgende destillative Depolymerisation des zunächst erhaltenen Oligo- bzw. Polycarbonats mit Struktureinheiten der Formel (I) wird im Temperaturbereich von 150-280°C, bevorzugt im Temperaturbereich von 190-250°C, besonders bevorzugt von 200°C-240°C, im Druckbereich von 0,01-20 mbar und in Gegenwart von Katalysatoren aus der Gruppe von Alkalien, Zinn-, Titanverbindungen, die in US 4 501 905 und US 4 440 937 angegeben sind, durchgeführt. Besonders bevorzugt wird die Depolymerisierung in Form einer Flash-Destillation durchgeführt, d.h. das aufgeschmolzene Oligo-Polycarbonat wird in einen erhitzten Reaktor dosiert und die entstehenden Monocarbonatdämpfe werden sofort kondensiert. Hierbei destilliert monomeres (I) ab, und es hinterbleibt ein Destillationsrückstand, der erfindungsgemäß mindestens teilweise, d.h. zu 50-100%, bevorzugt 60-100%, besonders bevorzugt 70-100% seiner Menge in die Umesterung von (II) mit (III) zurückgeführt wird.

Es kann vorteilhaft sein, den in die Umesterungsreaktion zurückzuführenden Destillationsrückstand aus der Depolymerisation vorab in der einzusetzenden Menge an (II) oder einem Teil davon aufzulösen und dann erst das Carbonat (III) zur Umesterung zuzusetzen. Dies erweist sich dann als zweckmäßig, wenn der Rückstand sehr hochmolekular oder aber ganz oder teilweise vernetzt ist. In der Regel jedoch ist diese Maßnahme nicht erforderlich. Die Umesterung kann dann im übrigen entsprechend den oben angegebenen Vorschriften vorgenommen werden.

Die Menge an Rückstand, die in die Umesterung zurückgeführt werden kann, richtet sich natürlich nach dessen Zusammensetzung und Qualität, die man durch Hydrolyse und gaschromatographische Analyse der Hydrolyseprodukte oder durch NMR-Spektroskopie ermitteln kann oder auch rein empirisch, indem man probeweise eine größere oder kleinere Menge des Rückstandes in die Umesterungsreaktion einsetzt und deren Einfluß auf die Depolymerisation und die Reinheit des erhaltenen Sechsringcarbonates untersucht. Der Fachmann wird auf diese Weise rasch die angemessene Menge an rückführbarem Rückstand ermitteln können.

Diese Art der Rückstandsverwertung ist besonders für die Synthese von solchen Sechsringcarbonaten interessant, deren Depolymerisation besonders anspruchsvoll ist und Anlaß zur Entstehung von größeren Mengen an Rückstand geben kann. Es sind dies insbesondere die Triole aus der Reihe der Trimethylolalkane und Pentaerythrit der Formeln 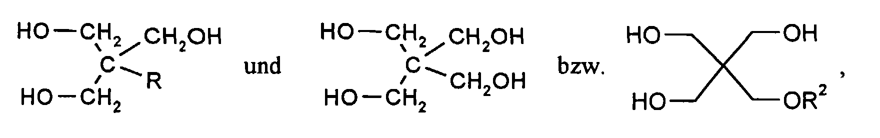 (II mit R¹ = CH₂OH) (II mit R = R¹ = CH₂OH),
da bei deren Depolymerisation leicht hochmolekulare oder gar vernetzte Strukturen entstehen können. Ganz besonders wichtig ist das erfindungsgemäße Verfahren für die Herstellung von Trimethylolpropan-Monocarbonat, aus dem neue hochwertige Lackrohstoffe hergestellt werden können, wie oben bereits ausgeführt wurde.

Erfindungsgemäß einsetzbare Säuren des Schwefels, deren Ester oder Salze für den Fall, daß eine Stabilisierung vorgesehen ist, sind solche der Formel 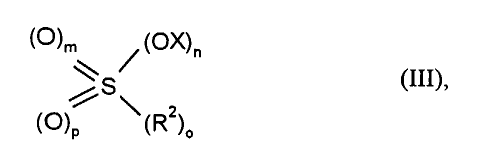 worin
die Indices m, n, o und p unabhängig voneinander die Ziffer Null oder Eins bedeuten, aber nur zwei dieser Indices gleichzeitig Null sein dürfen,
- X: für H^{⊕}, Li^{⊕}, Na^{⊕}, K^{⊕}, Rb^{⊕}, Cs^{⊕}, N(R³, R⁴, R⁵, R⁶)^{⊕}, worin R³ bis R⁶ unabhängig voneinander H, C₁-C₁₈-Alkyl, Phenyl oder Benzyl bedeuten, oder für C₁-C₁₈-Alkyl, C₆-C₁₂-Aryl oder Benzyl steht, wobei Alkyl, Aryl und Benzyl 1 - oder 2-mal durch Methyl, Ethyl, Chlor oder SO₃H substituiert sein kann, und
- R²: die Bedeutung von -OX, -OOH, -OSO₃, -OOSO₃, -NH₂, -NH(SO₃H), C₁-C₁₈-Alkyl oder C₆-C₁₂-Aryl, die in der angegebenen Weise substituiert sein können, annimmt, wobei für den Fall, daß R² OX bedeutet, nicht alle X eines der genannten Metallkationen bedeuten, und wobei R² für den Fall, daß n Null bedeutet, auch doppelt gebundenen Sauerstoff darstellen kann.

Solche Säuren des Schwefels sind beispielsweise schwefelige Säure, Schwefelsäure, ihre Anhydride SO₂ bzw. SO₃, aliphatische und aromatische Mono- und Polysulfonsäuren sowie Sulfen- und Sulfinsäuren mit 1 bis 18 C-Atomen, die partiell neutralisiert sein und demnach als Teil- oder Halbsalz vorliegen können, Peroxyschwefelsäure, Amidosulfonsäuren, Dischwefelsäure und Peroxydischwefelsäure und deren Halbsalze mit Alkalien, Ammoniak und Aminen, Aminomethansulfonsäuren und Hydroxymethansulfonsäuren, wie sie beispielsweise in Houben, Weyl Methoden der organ. Chemie, Band 9, S. 267, 281-3, 289-97 beschrieben sind.

In bevorzugter Weise ist in Formel (III) p=1, in besonders bevorzugter Weise sind p und m = 1, in ganz besonders bevorzugter Weise sind p, m und n = 1.

Erfindungsgemäß einsetzbare Säuren des Phsophors, deren Ester oder Salze für den Fall, daß eine Stabilisierung vorgesehen ist, sind solche der Formel 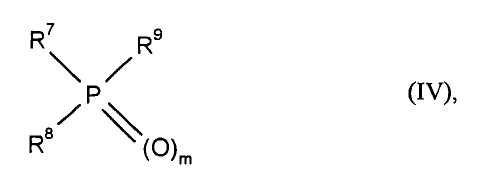 worin
- m: die Ziffer Null oder Eins bedeutet,
- R⁷: für C₁-C₂₀-(Cyclo)Alkyl, C₆-C₁₂-Aryl oder C₇-C₁₀-Aralkyl stehen, die 1- oder 2-fach durch O-C₁-C₄-Alkyl, S-C₁-C₄-Alkyl, COOH, CN, Cl, Br, NH₂ NH-C₁-C₄-Alkyl, N(C₁-C₄-Alkyl)₂, NH-C₆H₅, N(C₁-C₄-Alkyl-P(O)(OH)₂)₂ oder P(O)(OH)₂ oder zwei verschiedene von ihnen substituiert sein können und in deren (Cyclo)Alkylanteilen 1 oder 2 C-Atomen durch -O-, -S-, -NH-, -N(C₁-C₄-Alkyl)- oder -CO- ersetzt sein können und/oder 2-C-Atome durch eine Doppel- oder Dreifachbindung verknüpft sein können, und
- R⁸ und R⁹: unabhängig voneinander die Bedeutung OX haben, wobei X den oben im Zusammenhang mit Formel (III) angegebenen Bedeutungsumfang einschließlich der Einschränkung bezüglich der Metallkationen beim Vorhandensein von zwei X annimmt, wobei jedoch im Rahmen der Formel (IV) an die Stelle des Substituenten SO₃H der Substituent P(O)(OH)₂ tritt, und wobei weiterhin R⁸ den Bedeutungsumfang von R⁷ annehmen kann und R⁹ auch H bedeuten kann und wobei weiterhin R⁷ und R⁸ gemeinsam für -O-Arylen-O- oder -Arylen-O- stehen können, worin Arylen für Biphenylyl oder -C₆H₄-C₁-C₆-(Cyclo)alkylen-C₆H₄- steht.

Solche Säuren des Phosphors sind beispielsweise Verbindungen mit den charakteristischen Gruppen 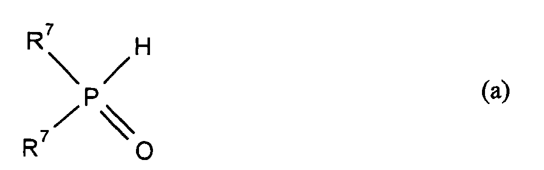 (vergl. Houben Weyl, Methoden der organischen Chemie, Bd. 12/1, S. 197), 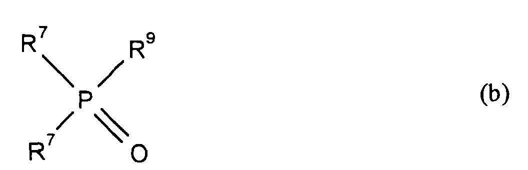 (ibid., S. 223, 227-8, 235, 238, 249, 255-7), 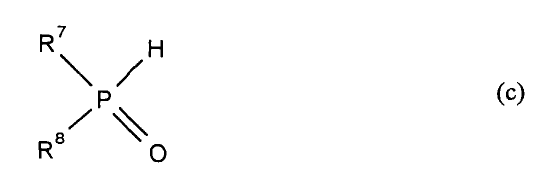 (ibid., S. 295, 322-3, 326-7, 329) oder 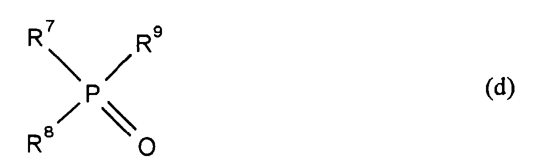 (ibid., S. 350, 355-6, 362, 365-6, 368-70, 374-82, 426-7, 444-5, 451-2, 466-73, 478-81, 485-7),
worin
R⁷, R⁸ und R⁹ die angegebene Bedeutung haben.

Die folgende Aufzählung zeigt, welche Verbindungen im Sinne der vorliegenden Erfindung als stabilisierend wirkende Säuren, Ester und Salze des Phosphors eingesetzt werden können, wobei die Bezeichnungen R, R', R¹ usw., A, X und andere nicht mit den obigen Substituentenbezeichnungen übereinstimmen, sondern den zitierten Schriften entnommen sind:
1) 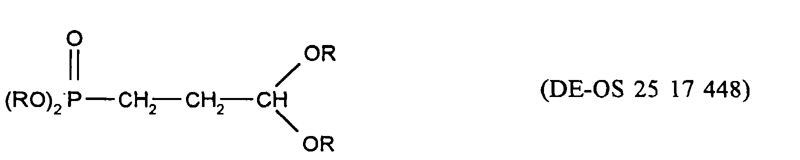
2) 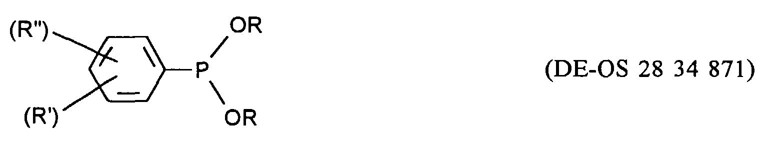
3) 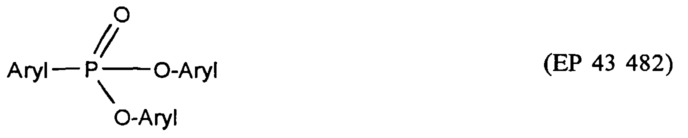
4) 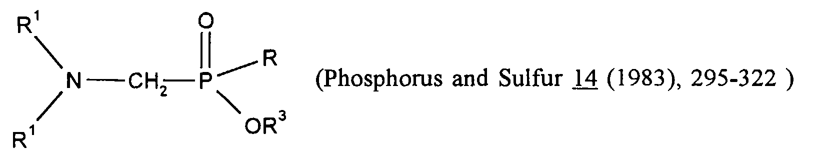
5) 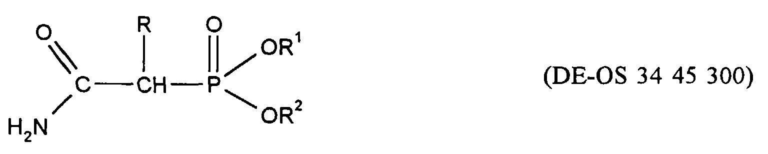
6) 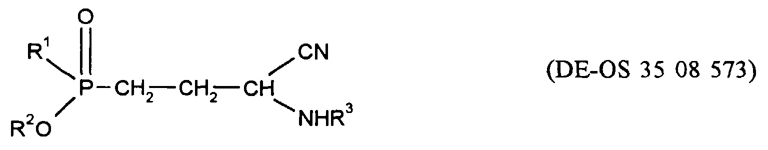
7) 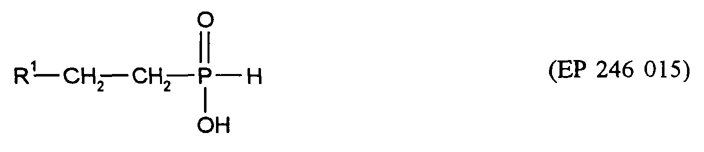
8) 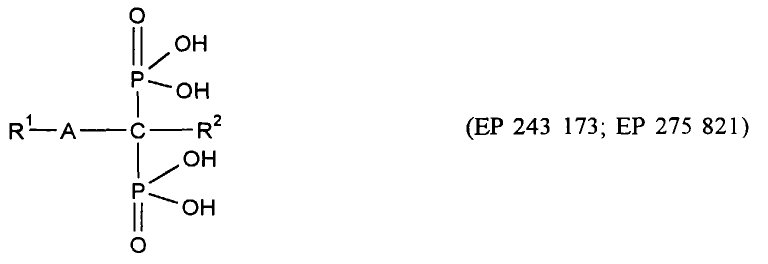
9) 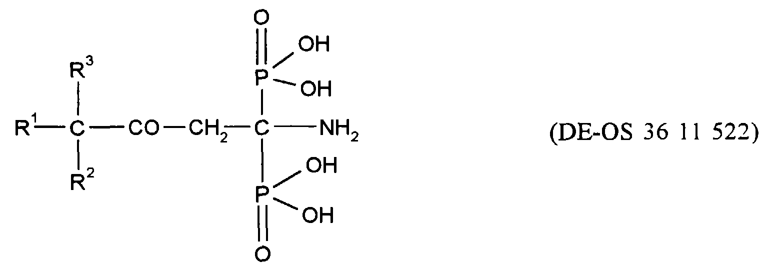
10) 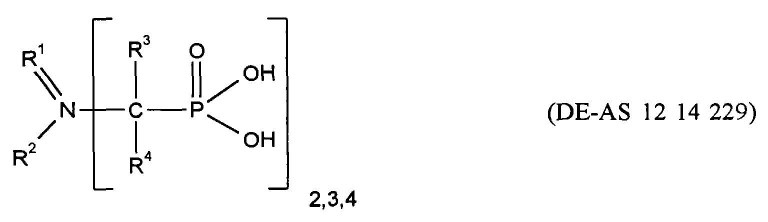
11) 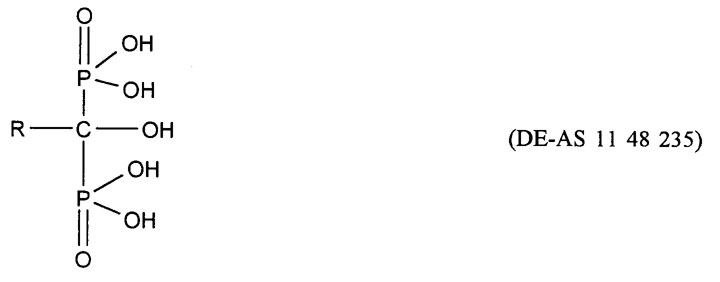
12) 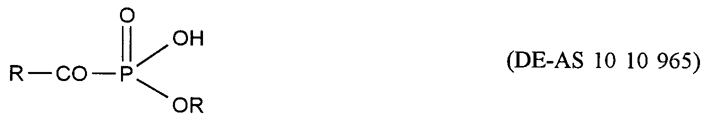
13) 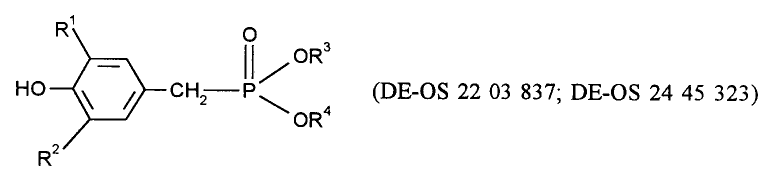
14) 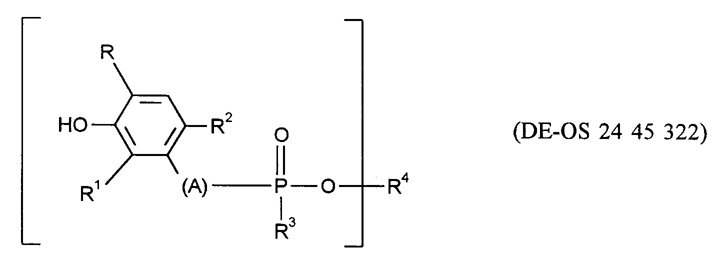
15) 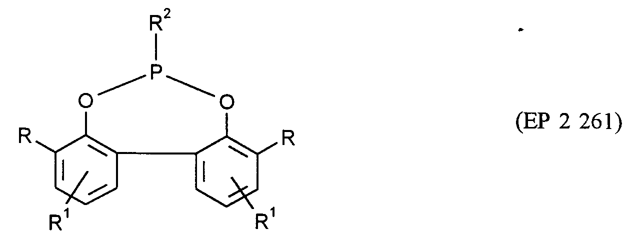
16) 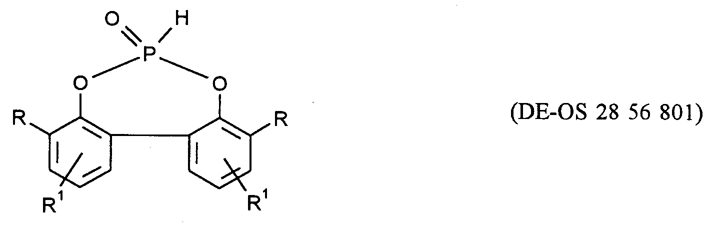
17) 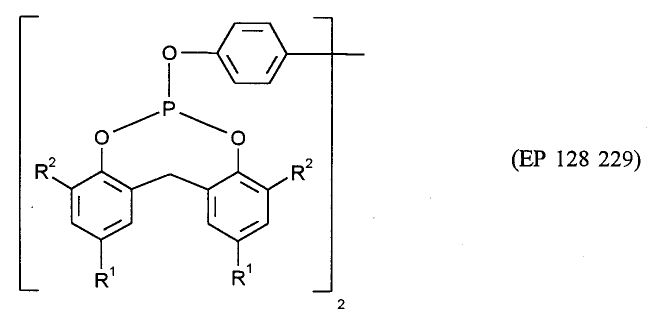
18) 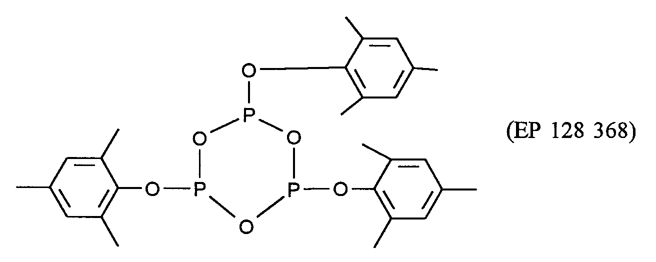
19) 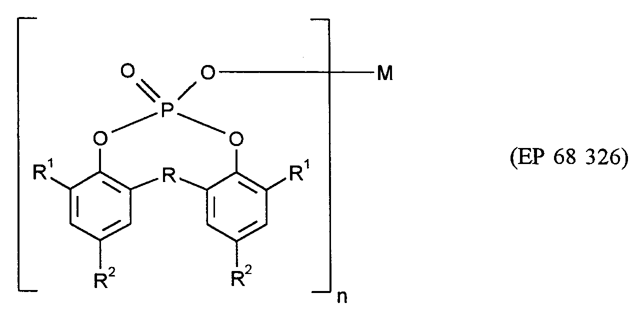
20) 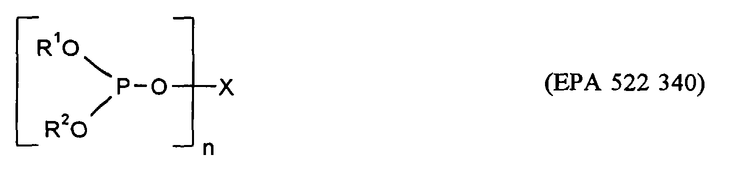
21) 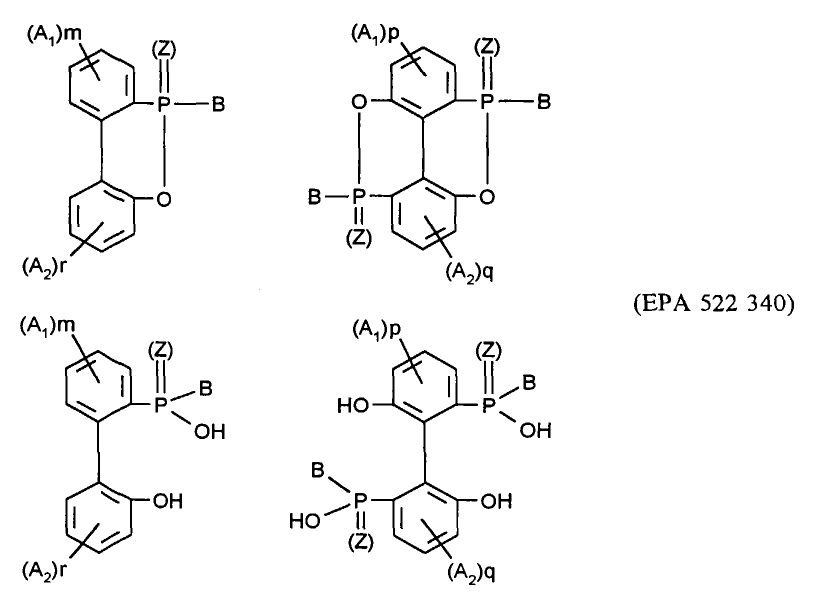
22) 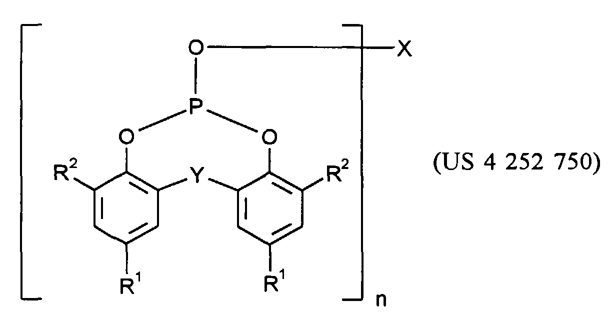
23) 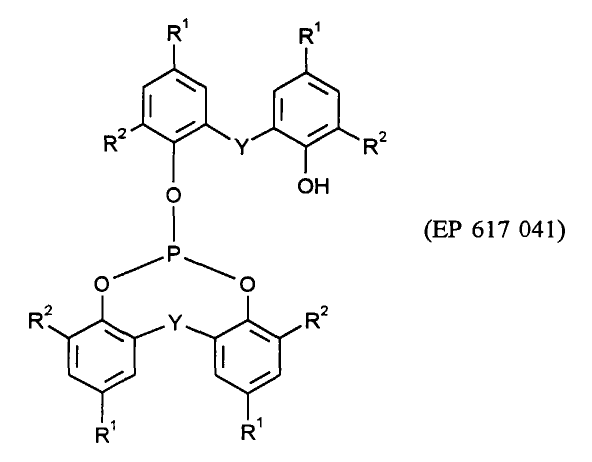
24) Unterphosphorige Säure, Phosphorige Säure, Phosphorsäure, die teil- und vollverestert und/oder teilweise mit Alkali-, Erdalkali- und Stickstoffbasen neutralisiert sein können (vergl. Houben Weyl, Bd. 12/2, S. 8, 24-5, 56-7, 61, 65-7, 173, 191-8, 209-10, 236, 250, 258, 260, 277, 309, 330-1).

Bevorzugt sind Verbindungen der Struktur 2), 4), 5), 7), 8) bis 13), 16), 17), 19) und 20) bis 24) besonders bevorzugt der Struktur 4), 8) bis 11), 17), 21) bis 24), ganz besonders bevorzugt der Struktur 10), 17), 21), 22) und 24).

In weiterhin bevorzugter Weise ist in Formel (IV) m = 1.

Von den aufgeführten Verbindungen des Schwefels und des Phosphors sind die des Phosphors bevorzugt. Eine beispielhafte und nicht erschöpfende Aufzählung von einzelnen Verbindungen ist die folgende: Dioctyl-phosphinigsäure, Dihexylphosphinigsäure, Dibenzylphosphinigsäure, Dicyclohexylphosphinigsäure, Diphenyl-phosphinigsäure, Bis-(4-chlorphenyl)-phosphinigsäure, Bis-(4-methoxyphenyl)-phosphinigsäure, Diethyl-phosphinsäure, Dipropyl-phosphinsäure, Diisopropyl-phosphinsäure, Dibutyl-phosphinsäure, Dihexyl-phosphinsäure, Dioctyl-phosphinsäure, Diphenyl-phosphinsäure, Bis-(4-methoxy-phenyl)-phosphinsäure, Dimethylphosphinsäure, Methyl-butyl-phosphinsäure, Methylphenyl-phosphinsäure, [3-Oxo-1,5-diphenyl-penten-(4)-yl-(1)]-phenylphosphinsäure, [4-Oxo-2-methyl-pentyl-(2)]-phenyl-phosphinsäure, [3-Oxo-1,5-diphenyl-propyl-(1)]-butyl-phosphinsäure, Phenyl-(3-chlor-phenyl)-phosphinsäure, Phenyl-(3-amino-phenyl)-phosphinsäure, Phenyl-(2-carboxy-phenyl)-phosphinsäure, Diethyl-phosphinsäure-ethylester, Dipropyl-phosphinsäure-propylester, Dibutyl-phosphinsäure-butylester, Ethyl-butyl-phosphinsäure-ethylester, Carbethoxymethyl-ethyl-phosphinsäure-ethylester, Butylbenzyl-phosphinsäure-ethylester, Methyl-phenyl-phosphinsäuremethylester, Ethan-phosphonigsäure, Propan-phosphonigsäure, 2-Methyl-propan-phospshonigsäure, Phenylmethan-phosphonigsäure, Triphenylmethan-phosphonigsäure, 2-Phenyl-ethylen-phosphonigsäure, Benzol-phosphonigsäure, 4-Methyl-benzol-phosphonigsäure, 4-Ethyl-benzol-phosphonigsäure, 2,4,6-Trimethyl-benzol-phosphonigsäure, 4-Chlor-benzol-phosphonigsäure, 4-Methoxy-benzol-phosphonigsäure, Naphthalin-1-phosphonigsäure, Ethan-phosphonigsaure-monomethylester, Benzol-phosphonigsäure-monobutylester, Benzol-phosphonigsäure-monobenzylester, Methan-phosphonigsäure-diphenylester, Ethan-phosphonigsäure-di-phenylester, Benzol-phosphonigsäure-di-phenylester, Naphthalin-1-phosphonigsäure-di-phenylester, 2-Phenyl-ethylen-phosphonsäure, 2,2-Diphenyl-ethylen-phosphonsäure, Methan-phosphonsäure, Ethan-phosphonsäure, Propan-1-phosphonsäure, Butan-1-phosphonsäure, Butan-2-phosphonsäure, Phenylmethan-phosphonsäure, (4-Methyl-phenyl)-methan-phosphonsäure, β-Naphthyl-methan-phosphonsäure, 2-Phenoxy-ethan-phosphonsäure, 4-Oxo-2-methyl-pentan-2-phosphonsäure, Diethylamino-methan-phosphonsäure, Diphosphono-methan-(methan-diphosphonsäure), 1,2-Diphosphono-ethan-(ethan-1,2-diphosphonsäure), 4-Oxo-2-methyl-pentan-2-phosphonsäure, 3-Oxo-2,5-dimethyl-cyclopentan-1-phosphonsäure, 3-Oxo-1-methyl-cyclohexan-1-phosphonsäure, Hydroxymethan-phosphonsäure, 1-Hydroxy-ethan-1-phosphonsäure, 1-Hydroxy-2-methyl-propan-1-phosphonsäure, α-Hydroxy-phenylmethan-phosphonsäure, 2-Hydroxy-propan-2-phosphonsäure, 1-Hydroxy-1-phenyl-ethan-1-phosphonsäure, α-Hydroxy-diphenylmethan-phosphonsäure, 1-Hydroxy-1-carboxy-ethan-1-phosphonsäure, 1-Hydroxy-cyclohexan-1-phosphonsäure, Benzol-phosphonsäure, 3-Methyl-benzol-phosphonsäure, 4-Methyl-benzol-phosphonsäure, 4-tert.-Butyl-benzol-phosphonsäure, Biphenyl-4-phosphonsäure, 2-Fluor-benzol-phosphonsäure, 4-Chlor-benzol-phosphonsäure, 4-Brom-benzol-phosphonsäure, 4-Fluor-benzol-phosphonsäure (Toluidinsalz), 2,3-Dichlor-benzol-phosphonsäure, 2,5-Dichlor-benzol-phosphonsäure, 2-Methoxy-benzol-phosphonsäure, 4-Methoxy-benzol-phosphonsäure, 4-Ethoxy-benzol-phosphonsäure, Propen-2-phosphonsäure, Phenoxy-methan-phosphonsäure, (Bis-[2-hydroxyethyl]-amino)-methan-phosphonsäure, 1,3-Bis-[N-phosphonomethyl-N-butylamino]-propan, 2-Methylamino-propan-2-phosphonsäure, Cyclohexan-phosphonsäure-diphenylester, Benzol-phosphonsäure-diphenylester, 2-Oxo-2-phenyl-4,5-benzo-1,3,2-dioxaphospholin, Ethylen-phosphonsäure-di-ethylester, Ethylen-phosphonsäure-di-phenylester, Propen-2-phosphonsäure-di-methylester, 3-Oxo-cyclohexan-phosphonsäure-diethylester, 3-Oxo-1,5,5-trimethyl-cyclohexan-phosphonsäure-di-butylester, 2-Cyan-ethan-phosphonsäure-dimethylester, 2-Carbomethoxy-ethan-phosphonsäure-dimethylester, 4-Oxo-pentan-2-phosphonsäure-di-methylester, 4-Oxo-2-methyl-pentan-2-phosphonsäure-dimethylester, 2-Oxo-heptan-4-phosphonsäure-di-butylester, 3-Oxo-1-phenyl-butan-1-phosphonsäure-dimethylester, 3-Oxo-1-phenyl-butan-1-phosphonsäure-di-ethylester, 1,2-Di-carbomethoxy-ethan-phosphonsäure-dimethylester, 1,2-Di-carboethoxy-propan-2-phosphonsäure-diethylester, 1-Amino-propan-1-phosphonsäure-diethylester, 1-Diethylamino-propan-1-phosphonsäure-diethylester, 1-Diethylamino-propen-(2)-1-phosphonsäure-diethylester

Die Mengen an o.a. Verbindungen, die für die Stabilisierung verwendet werden, betragen 0,001 bis 5 Gew.-%, bezogen auf das Gewicht des cyclischen Carbonats, vorzugsweise 0,005 bis 3, besonders bevorzugt 0,01 bis 2 Gew.-%.

Der Stabilisator kann direkt durch Eintragen in die Schmelze oder die Lösung des Carbonates im Carbonat verteilt werden oder indem man zunächst ein Masterbatch, ein Konzentrat des Stabilisators im Carbonat oder in einem Lösungsmittel, herstellt und dann dieses der zu stabilisierenden Menge des Carbonates in entsprechender Dosierung zufügt.

Geeignete Lösungsmittel für die Lösung des Carbonats und des Stabilisators sind inerte polare Lösungsmittel, wie Ether, Ester, Ketone, Nitrile, Amide, Lactame, Lactone. Genannt seien beispielsweise Essigsäureethyl- (oder -butyl-)ester, Kohlensäurediethylester, Butyrolacton, N-Methylpyrrolidon, Dimethylacetamid, Acetonitril und 2-Methoxy-propylacetat. Es kann auch ein Gemisch mehrerer der genannten Lösungsmittel eingesetzt werden. Diese Lösungsmittel haben eine Gesamt-C-Atomzahl von 2-20, bevorzugt 2-18, besonders bevorzugt 2-12. Lösungsmittel dieser Art sind dem Fachmann bekannt.

Die Mitverwendung eines Lösungsmittels ist vorteilhaft, weil dadurch die Polymerisationsneigung weiter verringert wird. Darum ist ein weiterer Gegenstand der vorliegenden Erfindung die Mitverwendung eines Lösungsmittels aus der Gruppe der oben genannten Lösungsmittel bei der Stabilisierung von Carbonaten. Bevorzugt verwendet werden Ester, Lactone, Amide und Lactame. Die Menge an Lösungsmittel liegt bei 2 bis 500 %, bevorzugt 3 bis 300 %, besonders bevorzugt 5 bis 200 % des Gesamtgewichts von Sechsringcarbonat und Stabilisator.

### Beispiel 1

In einem 4 l-Dreihalskolben mit Rührer, Thermometer und einer ca. 80 cm langen, verspiegelten Kolonne, die mit Raschigringen gefüllt war, wurden 1610 g (12 mol) Trimethylolpropan (TMP) und 2677 g (12,5 mol) Diphenylcarbonat (DPC) in Gegenwart von 2 g Dibutylzinnoxid bei 150 bis 160°C und 20 bis 30 mbar miteinander umgeestert und das entstehende Phenol abdestilliert. Nachdem die Phenolabspaltung beendet war, wurden die letzten Reste an Phenol über eine einfache Destillationsbrücke unter Einblasen von Stickstoff bei 2-30 mbar und 160 bis 165°C entfernt. Im Umesterungskolben blieben 1880 g TMP-Oligocarbonat.

Davon wurden kontinuierlich aus einem auf 150 bis 160° beheizten Tropftrichter 150 ml/h in einen 1 l-Dreihalskolben mit bodengängigem schnell laufendem Flügelrührer und Thermometer eingetragen. Der Kolben war rundum auf 220 bis 240° beheizt und auf 2 mbar evakuiert. Die durch die Oligomerspaltung entstehenden TMP-Carbonat-Dämpfe wurden über eine einfache Destillationsbrücke entnommen, sofort kondensiert und in einer Vorlage gesammelt. Während der kontinuierlichen Spaltung wurde von Zeit zu Zeit ein Teil des sich bildenden Sumpfes abgezogen.

Insgesamt wurden 1650 g des obigen TMP-Oligocarbonates in den Spaltungskolben eingetragen und in TMP-Carbonat übergeführt. Man erhielt 1351 g Destillat, das zu ca. 97 % aus TMP-Carbonat und 2 bis 3 % aus TMP bestand, und 286 g dickflüssigen Destillationssumpf. Dies entsprach einer Destillatausbeute von 82 %; der Verlust betrug 13 g. Der Rückstand enthielt 31,7 % CO₂ (Carbonatgruppe); für reines TMP-Carbonat errechnen sich 27,2 %. Der Rückstand löste sich beim Erhitzen in TMP.

### Beispiel 2

Beispiel 1 wurde wiederholt, wobei 1610 g TMP, 2636 g DPC und der Destillationsrückstand aus Beispiel 1 eingesetzt wurden. Der DPC-Anteil wurde gegenüber Beispiel 1 entsprechend dem erhöhten Carbonatgehalt des Rückstandes, der auch den Katalysator enthielt, verringert.

Die Umesterung wurde analog Beispiel 1 durchgeführt, wobei 2121 g TMP-Oligocarbonat entstanden. Dies wurde analog Beispiel 1 gespalten, wozu insgesamt 1954 g eingesetzt wurden. Man erhielt 1590 g Destillat und 356 g Destillationsrückstand. Dies entsprach einer Destillationsausbeute von etwa 81 % bei einem Verlust von 8 g. Das Destillat enthielt 97 % TMP-Carbonat und 2 bis 3 % TMP. Die Ausbeute für Beispiel 1 lag also knapp über 99 %, für beide Versuche über 90 %.

Damit wurde gezeigt, daß der Destillationsrückstand fast vollständig in TMP-Carbonat übergeführt werden kann. Bei immer erneutem Einsatz wird jedoch vorteilhafterweise je nach seiner Zusammensetzung ein mehr oder weniger großer Anteil des Rückstandes ausgeschleust und entsorgt. Die Gesamtausbeute an Destillat beträgt jedoch in jedem Fall deutlich über 95 %.

### Beispiel 3

In einer Apparatur analog Beispiel 1 wurden 1232,2 g (9,00 mol) Trimethylolpropan (TMP) 98 %ig, 2105,1 g (9,73 mol) Diphenylcarbonat 99 %ig in Gegenwart von 1,8 g Titantetrabutylat bei 150-160°C und einem Druck von 20 mbar miteinander umgeestert und das entstehende Phenol abdestilliert. Nachdem die Phenolabspaltung beendet war, wurden die letzten Reste an Phenol über einen einfachen Destillationskühler unter Einblasen von Stickstoff bei 2-20 mbar und einer Sumpftemperatur von 170°C entfernt.

Im Umesterungskolben blieben 1470 g TMP-Oligocarbonat.

1450 g des TMP-Oligocarbonats wurden kontinuierlich aus einem auf 160°C beheizten Tropftrichter mit einer Geschwindigkeit von 120 ml/h in einen 1 l Vierhalskolben mit bodengängigen schnell laufendem Flügelrührer und Thermometer eingetragen.

Der Spaltungskolben wurde bis an die Schliffansätze in ein auf 240°C beheiztes Ölbad eingetaucht und die Apparatur auf 0,5-1,5 mbar evakuiert. Die durch die Oligomerspaltung entstehenden TMP-Carbonat-Dämpfe wurden durch eine auf 240°C beheizte Vigreuxkolonne zu einem Destillationskühler geleitet, dort kondensiert und in einer Vorlage gesammelt. Nachdem etwa 700 g TMP-Oligocarbonat eingetropft worden waren, wurde die Flashdestillation unterbrochen und der Destillationssumpf noch heiß aus dem Kolben gegossen. Die restlichen Eduktmenge, ca. 750 g, wurde anschließend im gleichen Kolben der Flash-Destillation unterzogen.

Man erhielt ca. 1200 g öliges Destillat, das mit der Zeit kristallisierte. Das Produkt hatte folgende Zusammensetzung:
- TMP-Carbonat :: 92 %
- TMP :: 1,0 %
- 3-Ethyl-hydroxymethyl-oxetan:: 0,5 %
- Phenol :: < 0,3 %
- Rest :: Oligo-TMP-Carbonat

Das entsprach, bezogen auf den TMP-Einsatz, 76,6 % der theoretischen Ausbeute an cyclischen TMP-Carbonat.

### Beispiel 4 (Vergleich)

Jeweils 30 g kristallines, polymerfreies Trimethylolpropan-monocarbonat (TMP-C) (Fp. 41°C) wurden in Ampullen unter N₂ bei verschiedenen Temperaturen 48 h lang getempert und dann per Gelchromatographie die nicht polymerisierten Anteile an TMP-C gemessen und in % vom Edukt angegeben:
- 50°C:: 95 % nicht polymerisiertes TMP-C
- 65°C:: 87 % nicht polymerisiertes TMP-C
- 80°C:: 68 % nicht polymerisiertes TMP-C
- 100°C:: 29 % nicht polymerisiertes TMP-C

### Beispiele 5 bis 12

Beispiel 4 wurde wiederholt, aber in jede Ampulle wurden 0,03 g (∼ 0,1 Gew.-%) eines Stabilisators dem TMF-C zugemischt. Alle Ampullen wurden 48 h bei 100°C getempert und anschließend, wie in Beispiel 4 beschrieben, analysiert:

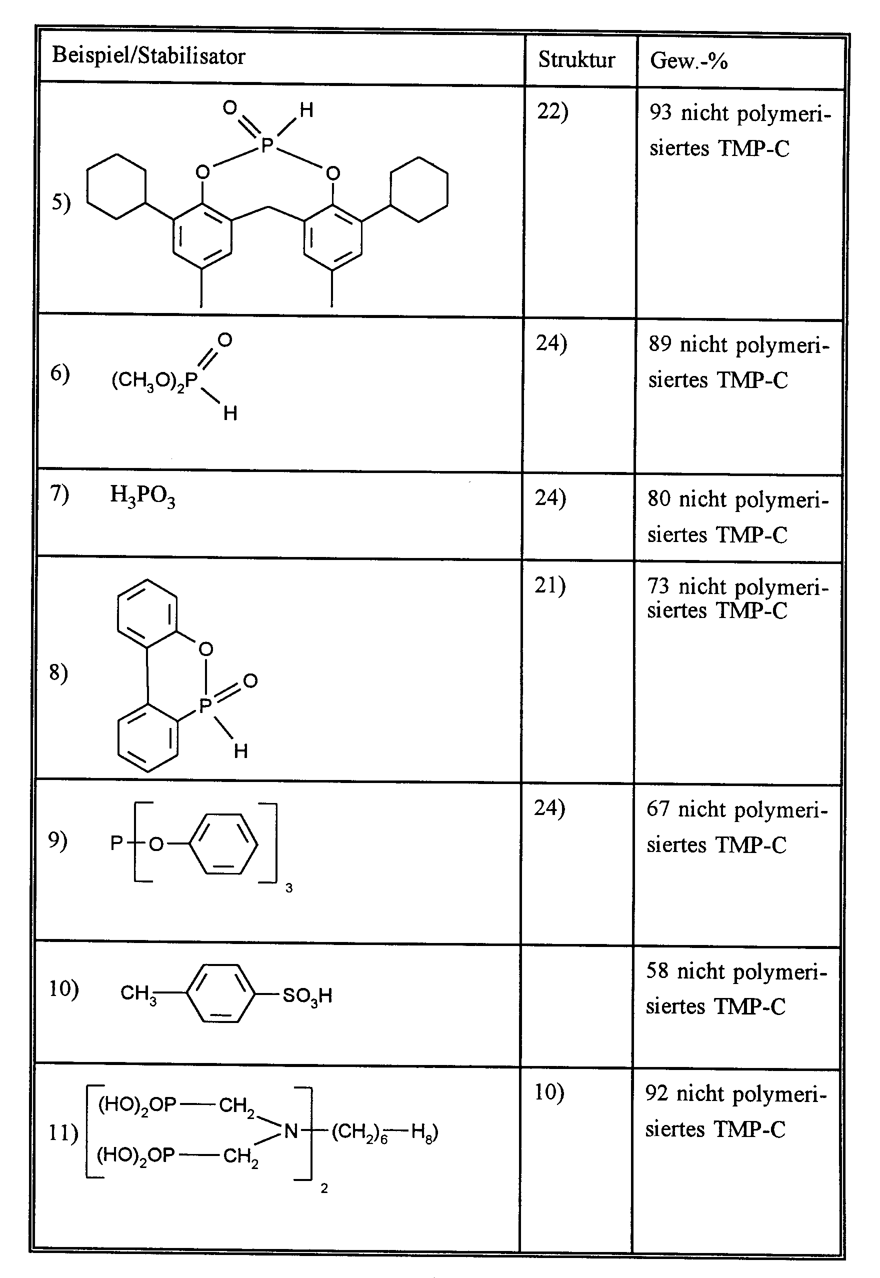

Man erkannt, daß die Polymerisation durch die Stabilisatoren deutlich gegenüber Beispiel 4 inhibiert wurde.

### Beispiele 12 bis 14

Beispiel 5 wurde wiederholt, aber der Anteil an Stabilisator im TMP-C und die Temperatur wurden verändert. Die Zeit (48 h) wurde beibehalten.

| 12) Stabilisator wie in Beispiel 11, 65°C | | | |
|---|---|---|---|
| Stabilisatormenge | 0,1 | 0,05 | 0,02 % |
| nicht polymer. TMP-C | 98 % | 97 % | 97 % |

| 13) Stabilisator wie in Beispiel 5, 65°C | | | |
|---|---|---|---|
| Stabilisatormenge | 0,1 | 0,05 | 0,02 % |
| nicht polymer. TMP-C | 97 % | 98 % | 97 % |

| 14) Stabilisator wie in Beispiel 6, 100°C | | | | |
|---|---|---|---|---|
| Stabilisatormenge | 0,5 | 0,2 | 0,1 | 0,05 % |
| nicht polymer. TMP-C | 98 % | 97 % | 85 % | 83 % |

Die verschiedenen Konzentrationen an Stabilisatoren gewähren einen guten Schutz vor unerwünschten Polymerisationen.

### Beispiele 15 bis 17

Je 30 g kristallines, polymerfreies TMF-C (Fp 41°C) wurden in 30 g Butylacetat (Beispiel 15), 30 g Methoxypropylacetat (Beispiel 16) bzw. 30 g N-Methylpyrrolidon (Beispiel 17) gelöst, mit je 0,1 % Stabilisator aus Beispiel 5 versetzt und 48 h bei 100°C analog Beispiel 5 getempert. Die stabilisierten Proben waren praktisch unverändert. In Vergleichsversuchen ohne Stabilisator sank der Monomeranteil auf 80 bis 85 %.

## Patentansprüche

1. Verfahren zur Herstellung von gegebenenfalls stabilisierten cyclischen Carbonaten der Formel (I) 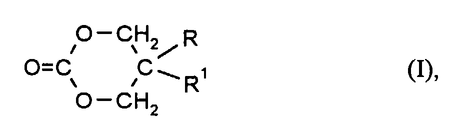 in der
R und R¹ gleich oder verschieden sind und H, geradkettiges oder verzweigtes C₁-C₆-Alkyl, C₃-C₇-Cycloalkyl oder -CH₂-OR² darstellen, worin R² für H, C₁-C₄-Alkyl, Allyl, Methallyl oder Benzyl steht und worin zwei R²-Gruppen gemeinsam -CH₂-, -CH(CH₃)-, -CH(C₂H₅)-, -CH(C₆H₅)- oder -CH(C₃H₇)- sein können, wobei R¹ zusätzlich C₆-C₁₂-Aryl bedeuten kann und wobei weiterhin R und R¹ gemeinsam mit dem C-Atom, das sie substituieren, einen Cyclopentan-, Cyclohexan-, Cycloheptan-, Oxetan-, Tetrahydrofuran-, Tetrahydropyran- oder Dioxanring bilden können,
durch Umesterung von 1,3-Propandiol-Verbindungen der Formel (II) 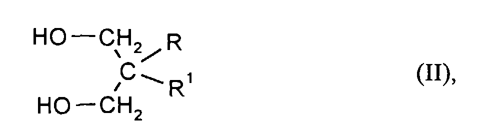 in der R und R¹ die oben angegebene Bedeutung haben,
mit Kohlensäureestern der Formel (III) 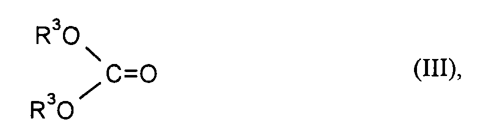 in der R³ C₁-C₄-Alkyl oder C₆-C₁₀-Aryl bedeutet,
unter Abspaltung von R³OH zu Oligo- oder Polycarbonaten und durch nachfolgende destillative Depolymerisation der so erhaltenen Oligo- oder Polycarbonate zu cyclischen Carbonaten (I), dadurch gekennzeichnet, daß man sowohl in der Stufe der Umesterung als auch in der nachfolgenden destillativen Depolymerisation in Gegenwart eines oder mehrerer Katalysatoren aus der Gruppe der Verbindungen von Zinn, Titan und Zirkon in einer Menge von 0,001 - 5 Gew.-%, bevorzugt in einer Menge von 0,03 -0,1 Gew.-%, bezogen auf die Gesamtmenge von (II) und (III), arbeitet, wobei zur Umesterung bei einer Temperatur von 120-180°C, bevorzugt von 150-170°C und zur Depolymerisation bei einer Temperatur von 150-280°C, bevorzugt 190-250°C, besonders bevorzugt von 200-240°C gearbeitet wird und die Temperatur zur Depolymerisation 30-150°C über der der Umesterung liegt, und man gegebenenfalls den bei der Depolymerisation erhaltenen Destillationsrückstand zu 50-100% seiner Menge in die Umesterungsreaktion von (II) mit (III) zurückführt, und wobei man dem cyclischen Carbonat (I) nach der Depolymerisation zur Stabilisierung gegen unerwünschte Polymerisation eine oder mehrere Verbindungen aus der Gruppe der Säuren des Schwefels und des Phosphors sowie deren Estern und Salzen in einer Menge von 0,001 - 5 Gew.-%, bezogen auf die Menge von (I), zusetzen kann.

2. Verfahren nach Anspruch 1, dadurch gekennzeichnet, daß an Stellen von R und R¹ die Reste R¹⁰ und R¹¹ treten, die gleich oder verschieden sind und H, CH₃, C₂H₅ oder -CH₂-OR² bedeuten, wobei R² den in Anspruch 1 gegebenen Umfang hat.

3. Verfahren nach Anspruch 1, dadurch gekennzeichnet, daß an Stelle von R³ der Rest R¹³ tritt, der Phenyl, Tolyl oder Chlorphenyl, bevorzugt Phenyl bedeutet.

4. Verfahren nach Anspruch 1, dadurch gekennzeichnet, daß man 60 bis 100%, bevozugt 70 bis 100% der Menge des Destillationsrückstandes in die Umesterung zurückführt.

5. Verfahren nach Anspruch 1, dadurch gekennzeichnet, daß eine Stabilisierung von cyclischen Carbonaten der Formel 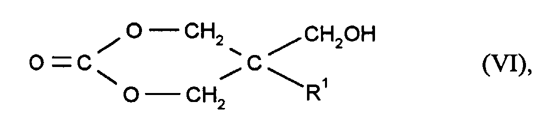 vorgenommen wid, in der
R¹ C₁-C₆-Alkyl, Cyclohexyl oder C₆-C₁₂-Aryl bedeutet.

6. Verfahren nach Anspruch 1, dadurch gekennzeichnet, daß man für den Fall der Stabilisierung den cyclischen Carbonaten neben den S- bzw. P-Verbindungen der genannten Art eines oder mehrere Lösungsmittel aus der Gruppe der Ether, Ester, Ketone, Nitrile, Amide, Lactame und Lactone, bevorzugt der Gruppe der Ester, Amide, Lactame und Lactone, in einer Menge von 2-500 %, bevorzugt 3-300 %, besonders bevorzugt 5-200 % des Gesamtgewichts von cyclischen Carbonaten und S- bzw. P-Verbindung zusetzt.

7. Verfahren nach Anspruch 1, dadurch gekennzeichnet, daß für den Fall der Stabilisierung als Säuren des Schwefels, deren Ester und Salze solche der Formel 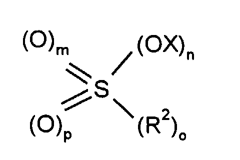 eingesetzt werden, worin
die Indices m, n, o und p unabhängig voneinander die Ziffer Null oder Eins bedeuten, aber nur zwei dieser Indices gleichzeitig Null sein dürfen,
X für H^{⊕}, Li^{⊕}, Na^{⊕}, K^{⊕}, Rb^{⊕}, Cs^{⊕}, N(R³, R⁴, R⁵, R⁶)^{⊕}, worin R³ bis R⁶ unabhängig voneinander H, C₁-C₁₈-Alkyl, Phenyl oder Benzyl bedeuten, oder für C₁-C₁₈-Alkyl, C₆-C₁₂-Aryl oder Benzyl steht, wobei Alkyl, Aryl und Benzyl 1- oder 2-mal durch Methyl, Ethyl, Chlor oder SO₃H substituiert sein kann, und
R² die Bedeutung von -OX, -OOH, -OSO₃, -OOSO₃, -NH₂, -NH(SO₃H), C₁-C₁₈-Alkyl oder C₆-C₁₂-Aryl, die in der angegebenen Weise substituiert sein können, annimmt, wobei für den Fall, daß R² OX bedeutet, nicht alle X eines der genannten Metallkationen bedeuten, und wobei R² für den Fall, daß n Null bedeutet, auch doppelt gebundenen Sauerstoff darstellen kann,
und als Säuren des Phosphors, deren Ester oder Salze solche der Formel 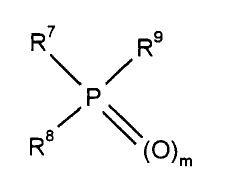 eingesetzt werden, worin
m die Ziffer Null oder Eins bedeutet,
R⁷ für C₁-C₂₀-(Cyclo)Alkyl, C₆-C₁₂-Aryl oder C₇-C₁₀-Aralkyl stehen, die 1- oder 2-fach durch O-C₁-C₄-Alkyl, S-C₁-C₄-Alkyl, COOH, CN, Cl, Br, NH₂ NH-C₁-C₄-Alkyl, N(C₁-C₄-Alkyl)₂, NH-C₆H₅, N(C₁-C₄-Alkyl-P(O)(OH)₂)₂ oder P(O)(OH)₂ oder zwei verschiedene von ihnen substituiert sein können und in deren (Cyclo)Alkylanteilen 1 oder 2 C-Atomen durch -O-, -S-, -NH-, -N(C₁-C₄-Alkyl)- oder -CO- ersetzt sein können und/oder 2-C-Atome durch eine Doppel- oder Dreifachbindung verknüpft sein können, und
R⁸ und R⁹ unabhängig voneinander die Bedeutung OX haben, wobei X den oben im Zusammenhang mit Formel (III) angegebenen Bedeutungsumfang einschließlich der Einschränkung bezüglich der Metallkationen beim Vorhandensein von zwei X annimmt, wobei jedoch im Rahmen der Formel (IV) an die Stelle des Substituenten SO₃H der Substituent P(O)(OH)₂ tritt, und wobei weiterhin R⁸ den Bedeutungsumfang von R⁷ annehmen kann und R⁹ auch H bedeuten kann und wobei weiterhin R⁷ und R⁸ gemeinsam für -O-Arylen-O- oder -Arylen-O- stehen können, worin Arylen für Biphenylyl oder -C₆H₄-C₁-C₆-(Cyclo)alkylen-C₆H₄- steht.

8. Verfahren nach Anspruch 1, dadurch gekennzeichnet, daß den cyclischen Carbonaten für den Fall der Stabilisierung mindestens eine Verbindung aus der Gruppe der Säuren des Phosphors sowie deren Estern und Salzen zusetzt.

9. Gemische von cyclischen Carbonaten der Formel (I) mit mindestens einer Verbindung aus der Gruppe der Säuren des Schwefels und des Phosphors sowie deren Ester oder Salzen in Mengen von 0,001 bis 5 Gew.-% der genannten Schwefel- bzw. Phosphorverbindungen, bezogen auf die Mengen der cyclischen Carbonate, sowie gegebenenfalls mit einem zusätzlichen Gehalt an einem oder mehreren Lösungsmitteln aus der Gruppe der Ether, Ester, Ketone, Nitrile, Amide, Lactame oder Lactone, bevorzugt der Gruppe der Ester, Amide, Lactame und Lactone, in einer Menge von 2 bis 500 %, bevorzugt 3 bis 300 %, besonders bevorzugt 5 bis 200 % des Gesamtgewichts von cyclischen Carbonaten und S- bzw. P-Verbindung.
